(19) Europäisches Patentamt
European Patent Office
Office européen des brevets

(11) Veröffentlichungsnummer: **0 071 064**
**B1**

# (12) EUROPÄISCHE PATENTSCHRIFT

(45) Veröffentlichungstag der Patentschrift: **29.05.85**

(51) Int. Cl.⁴: **C 07 C 141/02**

(21) Anmeldenummer: **82106200.7**

(22) Anmeldetag: **10.07.82**

(54) **Verfahren zur Herstellung von alpha, omega-Bis-fluorsulfato-perfluoralkanen.**

(30) Priorität: **16.07.81 DE 3128118**

(43) Veröffentlichungstag der Anmeldung: **09.02.83 Patentblatt 83/6**

(45) Bekanntmachung des Hinweises auf die Patenterteilung: **29.05.85 Patentblatt 85/22**

(84) Benannte Vertragsstaaten: **BE CH DE FR GB IT LI NL**

(56) Entgegenhaltungen:
**CHEMICAL ABSTRACTS, Band 77, Nr. 25, 18. Dezember 1972, Seite 367, Nr. 164122g, Columbus, Ohio, USA C.G. KRESPAN: "Synthesis and pyrolysis of fluorosulfates"**
**JOURNAL OF THE AMERICAN CHEMICAL SOCIETY, Band 83, 20. November 1961, Seiten 4521-4525, J.M. SHREEVE et al.: "Some reactions of peroxydisulfuryl difluoride"**
**CHEMICAL ABSTRACTS, Band 93, Nr. 25, 22. Dezember 1980, Seite 794, Nr. 238771x, Columbus, Ohio, USA M.A. KURYKIN et al.: "Reaction of radical addition to delta 2,3-perfluoroolefins"**
**INORGANIC CHEMISTRY, Band 3, Nr. 2, February 1964, Seiten 287-288, Washington, USA M. LUSTIG et al.: "Preparation of an acyl fluoride from an O-fluorosulfate; Some new O-fluorosulfates"**

(73) Patentinhaber: **HOECHST AKTIENGESELLSCHAFT, Postfach 80 03 20, D-6230 Frankfurt am Main 80 (DE)**

(72) Erfinder: **Millauer, Hans, Dr., An den sieben Bäumen 21b, D-6236 Eschborn (DE)**

**EP 0 071 064 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

α,ω-Bis-fluorsulfato-perfluoralkane sind Verbindungen der allgemeinen Formel

$$FSO_2-O-R_f-O-SO_2F\ ,$$

worin $R_f$ = perfluorierter verzweigter oder unverzweigter Alkylenrest.

Sie sind wertvolle Zwischenprodukte auf verschiedenen Sachgebieten, insbesondere auf dem Polymerengebiet.

Wenn die beiden Enden des perfluorierten Alkylenrestes $-R_f-$ jeweils durch eine $-CF_2$-Gruppe gebildet werden (wenn also $R_f = CF_2-\ldots-CF_2$), kommt man – ausgehend von solchen entsprechenden α,ω-Bis-fluorsulfato-perfluoralkanen – zu Polymeren bzw. Polymerenbausteinen auf folgendem Weg.

Aus den α,ω-Bis-fluorsulfato-perfluoralkanen werden zunächst – etwa durch Zersetzung in Gegenwart von Cäsiumfluorid CsF als Katalysator [vgl. J. Fluorine Chemistry 16 (1980), S. 63 bis 73, insbesondere S. 65, Abs. 2] – die beiden Fluorsulfatogruppen unter Bildung zweier Säurefluoridgruppen abgespalten:

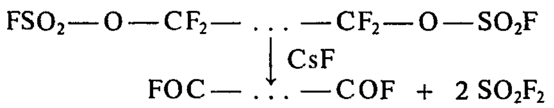


Die dabei entstehenden Perfluoralkan-α,ω-dicarbonsäuredifluoride können dann als solche oder auch nach Umwandlung in die entsprechenden freien Dicarbonsäuren oder deren Ester als Monomere für die Herstellung von Polyestern oder Polyamiden mit interessanten anwendungstechnischen Eigenschaften verwendet werden.

Die Perfluoralkan-α,ω-dicarbonsäuredifluoride können aber auch nur einseitig verestert werden, was etwa nach dem in der DE-OS 2 751 050 beschriebenen Verfahren geschehen kann. Dieses Verfahren läuft jedoch nicht bzw. nur wenig selektiv zu den perfluorierten Dicarbonsäurefluoridestern ab, sondern führt stets zu Gemischen mit den schwer abtrennbaren Ausgangsprodukten sowie Diestern. Die Dicarbonsäurefluoridester können (nach ihrer ziemlich aufwendigen Abtrennung) dann nach bekannten Methoden in perfluorierte Vinylether mit auch noch einer Estergruppe etwa nach folgendem Reaktionsschema umgewandelt werden:

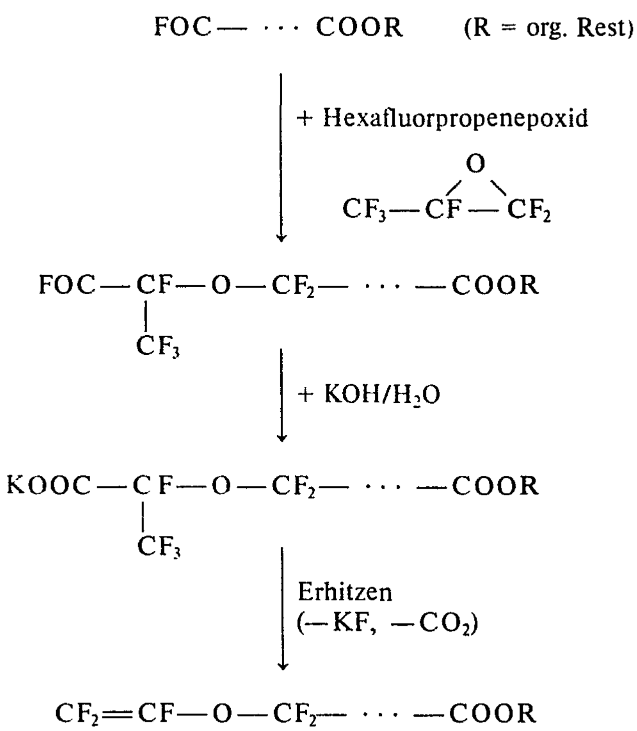


Die perfluorierten Vinylether mit noch einer Estergruppe sind wichtige Monomere für die Polymerisation bzw. Copolymerisation mit anderen Fluorolefinen (wie z. B. mit Tetrafluorethylen) zwecks Herstellung von Ionen-selektiven Membranen, Kationenaustauschermassen und Fluorelastomeren.

Eine als Polymerenbaustein bzw. -ausgangssubstanz besonders bedeutende perfluorierte Dicarbonsäure ist die Perfluorbernsteinsäure bzw. deren Difluorid oder Fluoridester. Das Fluorsulfato-Vorprodukt der Perfluorbernsteinsäure und deren genannter Derivate ist das 1,4-Bis-fluorsulfatoperfluorbutan:

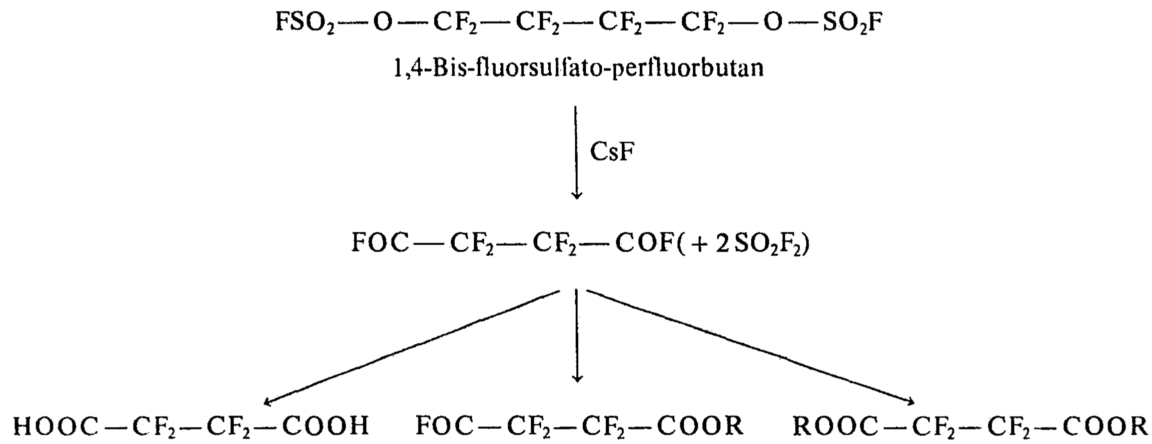


Daher ist von den $\alpha,\omega$-Bis-fluorsulfato-perfluoralkanen auch das 1,4-Bis-fluorsulfato-perfluorbutan von besonderer Bedeutung.

Zur Herstellung von $\alpha,\omega$-Bis-fluorsulfato-perfluoralkanen sind verschiedene Methoden bekannt.

Eine Methode zur Herstellung etwa des 1,2-Bis-fluorsulfatotetrafluorethans wird beschrieben von J. M. Shreeve und G. H. Cady in J. Am. Chem. Soc. 83, 4521 ff., insbesondere 4523 (1961). Die Methode besteht in der Umsetzung von Tetrafluorethylen mit Peroxodisulfuryl-difluorid offensichtlich nur in der Gasphase:

$$CF_2{=}CF_2 + FSO_2{-}O{-}O{-}SO_2F \rightarrow FSO_2{-}O{-}CF_2{-}CF_2{-}O{-}SO_2F$$

Die Autoren weisen besonders darauf hin, daß die Reaktion nur bei niedrigen Tetrafluorethylen-Drucken und -Konzentrationen sowie einem immer aufrechtzuerhaltenden Überschuß an Peroxodisulfuryl-difluorid funktioniert, da anderenfalls — also etwa bei höheren Tetrafluorethylen-Konzentrationen — infolge der radikalbildenden Wirkung des Peroxodisulfuryl-difluorids hauptsächlich nur Polymerisation des Tetrafluorethylens stattfindet. In der Beschreibung des Versuchsergebnisses wird als einziges Reaktionsprodukt 1,2-Bis-fluorsulfato-tetrafluorethan — ohne einen Hinweis auf die Ausbeute — neben nicht identifizierten polymeren Feststoffen sowie geringeren Mengen Carbonyldifluorid und Pyrosulfurylfluorid angegeben.

1,4-Bis-fluorsulfato-perfluorbutan und dessen Herstellung ist bekannt aus dem in Tetrahedron Vol. 37, S. 487 bis 491 veröffentlichten Artikel von A. Germain und A. Commeyras. Die Herstellung erfolgt durch Elektrolyse (anodische Oxidation) von 1,4-Bis-jod-perfluorbutan in einem Gemisch aus Fluorsulfonsäure und einem Alkalifluorsulfonat. Die Autoren sind der Meinung, daß ein direkter Elektrodenprozeß vorliegt, schließen aber einen gleichzeitigen, indirekten Prozeß über »$I^+$« nicht aus (S. 488, rechte Spalte).

Als Ausbeute ist in Tabelle 1 auf S. 489 des Artikels von A. Germain und A. Commeyras 70% angegeben. Obwohl diese Ausbeute relativ hoch liegt, ist das Verfahren insgesamt wegen der nicht ganz einfachen Zugänglichkeit des Ausgangsproduktes 1,4-Bis-jod-perfluorbutan nicht ganz befriedigend. Das 1,4-Bis-jod-perfluorbutan wird nämlich bei der Umsetzung von Tetrafluorethylen mit Jod nur in schlechter Ausbeute neben dem Hauptprodukt 1,2-dijodtetrafluorethan gebildet.

Ansonsten ist die Methode von A. Germain und A. Commeyras wohl auch auf die Herstellung anderer $\alpha,\omega$-Bis-fluorsulfatoperfluoralkane (aus den entsprechenden $\alpha,\omega$-Bis-jod-perfluoralkanen) anwendbar.

Die Darstellung eines $\alpha,\omega$-Bis-fluorsulfato-perfluoralkans mit einer verzweigten Perfluoralkankette beschreibt C. G. Krespan in J. Fluorine Chemistry 2, S. 173 bis 179 (1972/73). In einer Gasphasenreaktion (ähnlich der von J. M. Schreeve und G. H. Cady beschriebenen, siehe a. a. O.) wird das Olefin — hier: Hexafluorpropen — mit Peroxydisulfuryl-difluorid bei Raumtemperatur oder leicht darüber erhöhter Temperatur umgesetzt. Dabei soll das 1 : 1-Addukt 1,2-Bis-fluorsulfatohexafluorpropan mit einer Ausbeute von 62% neben 22% des 2 : 1-Addukts entstehen:

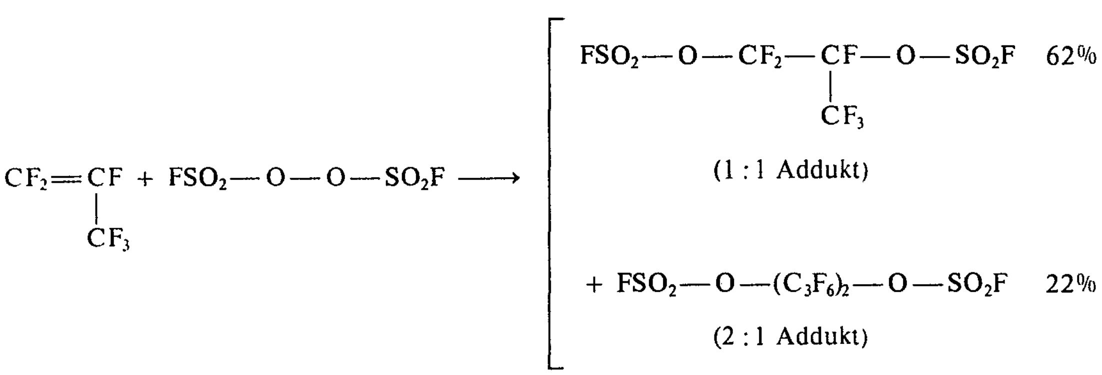

Über die Isomerenverteilung in dem 2 : 1-Addukt sind keine weiteren Angaben gemacht.

Wegen der erheblichen Bedeutung gerade der 2 : 1-Addukte aus Perfluorolefinen und Peroxodisulfuryl-difluorid, insbesondere des 1,4-Bis-fluorsulfato-perfluorbutans (= 2 : 1-Addukt aus Tetrafluorethylen und Peroxodisulfuryl-difluorid), als Zwischenprodukte für die Herstellung der entsprechenden Perfluordicarbonsäuren und deren Derivaten etc. (siehe die Beschreibungseinleitung), sowie wegen der bisher nur ziemlich unbefriedigenden Synthesemethoden für die genannten 2 : 1-Addukte bestand die Aufgabe, ein verbessertes Verfahren zu deren Herstellung — insbesondere zur Herstellung des 1,4-Bis-fluorsulfato-perfluorbutans — zu finden.

Diese Aufgabe konnte erfindungsgemäß durch Umsetzung von perfluorierten $\alpha$-Olefinen mit Peroxodisulfuryl-difluorid in flüssiger Phase gelöst werden; dabei ist allerdings die Konzentration des Peroxodisulfuryl-difluorids in der flüssigen Phase innerhalb eines bestimmten Konzentrationsbereiches im wesentlichen konstant zu halten.

Erfindungsgegenstand ist somit ein Verfahren zur Herstellung von $\alpha,\omega$-Bis-fluorsulfato-perfluoralkanen durch Umsetzung von perfluorierten $\alpha$-Olefinen mit Peroxodisulfuryl-difluorid $FSO_2O—OSO_2F$, das dadurch gekennzeichnet ist, daß man die perfluorierten $\alpha$-Olefine in eine das Peroxodisulfuryl-difluorid enthaltende flüssige Phase einleitet, wobei man die Konzentration des Peroxodisulfuryl-difluorids in der flüssigen Phase innerhalb des Konzentrationsbereiches von etwa 0,005—0,2, vorzugsweise von etwa 0,01—0,1 Mol/l, im wesentlichen konstant hält.

Die gewünschten 2 : 1-Addukte aus Perfluorolefinen und Peroxodisulfuryl-difluorid entstehen hier — weitgehend unabhängig von den Verfahrensbedingungen innerhalb der angegebenen Grenzen — in hoher Selektivität und Ausbeute neben geringeren Mengen auch der 1 : 1-, 3 : 1-, 4 : 1- und gegebenenfalls noch 5 : 1-Addukte. Dieses Ergebnis war außerordentlich überraschend, denn aufgrund der Veröffentlichungen von J. M. Schreeve und H. Cady (a. a. O.) sowie von C. G. Krespan (a. a. O.) war kaum anzunehmen, daß die Reaktion von Perfluorolefinen mit Peroxodisulfuryl-difluorid in Richtung auf die Entstehung der entsprechenden 2 : 1-Addukte als Hauptprodukte zu lenken ist. Aufgrund der Veröffentlichung von J. M. Schreeve und G. H. Cady mußte man nämlich davon ausgehen, daß das Perfluorolefin wegen der Polymerisations-auslösenden Wirkung des Peroxodisulfuryl-difluorids überhaupt nur in sehr niedriger Konzentration mit dem Peroxodisulfuryl-difluorid zu einem Addukt — und dann nur zu dem 1 : 1-Addukt (1,4-Bis-fluorsulfato-perfluorbutan) — umzusetzen ist. Bei einer höheren Perfluorolefin-Konzentration war nach dieser Literaturstelle die Bildung von festen Tetrafluorethylen-Polymerisaten zu erwarten.

Nach der Veröffentlichung von C. G. Krespan entsteht neben dem 1 : 1-Addukt als Hauptprodukt (62%) zwar auch noch 2 : 1-Addukt, jedoch nur als Nebenprodukt (22%) — und dies nicht mit Tetrafluorethylen, sondern mit Hexafluorpropen als Ausgangs-perfluorolefin.

Die erfindungsgemäße Führung der Reaktion in Richtung auf das 2 : 1-Addukt als überwiegendes Hauptprodukt dürfte durch die Ausführung der Umsetzung in flüssiger Phase (J. M. Schreeve und G. H. Cady sowie C. G. Krespan: in der Gasphase!) unter ganz bestimmten Konzentrationsverhältnissen bedingt sein.

Als perfluorierte $\alpha$-Olefine werden für das erfindungsgemäße Verfahren Verbindungen der Formel

$$CF_2{=}CF{-}R_f$$

worin $R_f$ = F oder Perfluoralkyl mit 1—8 C-Atomen, vorzugsweise F oder $CF_3$, insbesondere F, bedeutet, eingesetzt. Beispielhafte derartige perfluorierte $\alpha$-Olefine sind Tetrafluorethylen, Hexafluorpropen, Octafluor-n-buten-1, Hexafluor-i-penten-1 etc., wobei Tetrafluorethylen und Hexafluorpropen — insbesondere Tetrafluorethylen — besonders bevorzugt sind. Diese Perfluorolefine sind nach bekannten Methoden erhältlich und z. T. auch Handelsprodukte.

Das Peroxodisulfuryl-difluorid $FSO_2—O—O—SO_2F$ ist ebenfalls nach bekannten Verfahren — also etwa durch direkte Reaktion von $SO_3$ und Fluor in Gegenwart eines $Ag_2F_2$-Katalysators, durch Oxidation von Metallfluorsulfaten mit Fluor oder auch durch anodische Oxidation von Lösungen von Alkalifluorsulfaten in Fluorsulfonsäure — herstellbar (siehe F. B. Dudley, J. Chem. Soc. 1963, S. 3407—3411).

Bei der Durchführung des erfindungsgemäßen Verfahrens wird das Perfluorolefin in das in einem inerten Lösungsmittel gelöste Peroxodisulfuryl-difluorid mit einer solchen Geschwindigkeit eingeleitet, daß möglichst kein oder jedenfalls nicht allzu viel unreagiertes Perfluorolefin verbleibt.

Als inerte Lösungsmittel können z. B. perfluorierte Kohlenwasserstoffe, Fluorsulfonsäure $FSO_3H$ sowie auch die zur elektrochemischen Herstellung von Peroxodisulfuryl-difluorid verwendeten Lösungen von Alkalifluorsulfonaten in Fluorsulfonsäure, die bei der erfindungsgemäßen Umsetzung entstehenden $\alpha,\omega$-Bis-fluorsulfato-perfluoralkane selbst etc. verwendet werden.

Die Reaktionstemperatur kann im Prinzip in einem ziemlich weiten Bereich — im allgemeinen zwischen etwa −20 und etwa +100°C — gewählt werden; vorzugsweise liegt sie aber zwischen etwa 0 und etwa 50°C.

Da der Siedepunkt des Peroxodisulfuryl-difluorids bei etwa 65°C liegt, kann die Reaktion oberhalb dieser Temperatur bei Normaldruck natürlich nur dann durchgeführt werden, wenn ein entsprechend höher siedendes inertes Lösungsmittel verwendet wird.

Obwohl im Prinzip unter- oder überatmosphärischer Druck möglich sind, ist jedoch Normaldruck schon aus wirtschaftlichen Gründen eindeutig bevorzugt.

Außer der Durchführung der Umsetzung in flüssiger Phase ist für das Gelingen der Reaktion wesentlich und kritisch, daß die Konzentration des Peroxodisulfuryl-difluorids in der flüssigen Phase innerhalb des vorstehend angegebenen Konzentrationsbereichs — und darin wiederum im wesentlichen konstant — gehalten wird. Wegen des im Laufe der Reaktion erfolgenden Verbrauchs an Peroxodisulfuryl-difluorid muß dieses deswegen laufend zugeführt werden. Die Kontrolle der Konzentration des Peroxodisulfuryl-difluorids in der flüssigen Phase kann auf bekannte Weise — etwa durch Probeentnahme und Titration — geschehen.

Ebenso auf bekannte Weise — z. B. durch Destillation — ist auch die Aufarbeitung des Reaktionsgemisches möglich.

Die Reaktion kann sowohl kontinuierlich als auch diskontinuierlich durchgeführt werden.

Eine bevorzugte Ausführungsform des erfindungsgemäßen Verfahrens besteht darin, die perfluorierten $\alpha$-Olefine in die flüssige Phase einer Elektrolysezelle einzuleiten, in welcher durch Elektrolyse einer Lösung von Alkalifluorsulfonat in Fluorsulfonsäure Peroxodisulfuryl-difluorid gebildet und im Ausmaß von dessen Verbrauch laufend nachgeliefert wird. In diesem Fall ist die Alkalifluorsulfonat-Lösung in Fluorsulfonsäure auch gleich das Lösungsmittel für das Peroxodisulfuryl-difluorid, und die Lösung des Alkalifluorsulfonats und des Peroxodisulfuryl-difluorids in Fluorsulfonsäure stellt die flüssige Phase des erfindungsgemäßen Verfahrens dar.

Die Durchführung des Verfahrens als derartiger »in cell«-Prozeß ist bei Verwendung elektrochemischer Zellen etwa in Topf- oder Trogform, die bevorzugt im Labor oder im kleineren technischen Maßstab angewandt werden, besonders bequem und einfach. Die elektrochemischen Zellen können geteilte oder ungeteilte Zellen sein. Im allgemeinen ist eine ungeteilte Zelle völlig ausreichend. Ihrer einfacheren und billigeren Bauweise steht allerdings eine geringfügig niedere Stromausbeute gegenüber, welche durch die kathodische Reduktion des Peroxodisulfuryl-difluorids bedingt wird.

In einer geteilten Zelle können zur Abteilung der Elektrodenräume poröse Diaphragmen wie z. B. Glasfritten oder poröses Polytetrafluorethylen benutzt werden.

Als Elektrodenmaterialien kommen die für die elektrochemische Erzeugung von Peroxodisulfuryl-difluorid bekannten Anoden- und Kathodenmaterialien in Frage. Geeignet sind daher z. B. Platin und Platinlegierungen (wie Platin-Iridium-Legierungen etc.). Bevorzugtes Elektrodenmaterial ist glasartiger Kohlenstoff (glassy carbon).

Der Elektrolyt besteht aus Fluorsulfonsäure, in welcher zwecks Verbesserung der elektrischen Leitfähigkeit ein Salz — vorzugsweise ein Alkalisalz — der Fluorsulfonsäure gelöst ist. Besonders bevorzugte derartige Leitsalze sind Li-, Na- und K-Fluorsulfat.

Zur Herstellung der Elektrolytlösung geht man zweckmäßig von einem Alkalichlorid oder -bromid aus, das in einer Konzentration von etwa 0,05 bis etwa 5 m, vorzugsweise von etwa 0,1 bis etwa 1 m, in Fluorsulfonsäure gelöst wird. Der dabei freigesetzte Chlor- bzw. Bromwasserstoff entweicht aus der Lösung und wird durch Ausblasen der Elektrolytlösung z. B. mit Stickstoff vollständig entfernt. Auf diese Weise hergestellte Elektrolytlösungen können ohne weitere Vorbehandlung für die Elektrolyse eingesetzt werden.

Mit, oder besser nach dem Beginn der Elektrolyse — wenn die gewünschte Peroxodisulfuryl-difluorid-Konzentration erreicht ist — wird dann das Perfluorolefin in die Elektrolytphase eingeleitet. Jedenfalls im Falle des Einleitens der gasförmigen Perfluorolefine — insbesondere des Tetrafluorethylens — ist es zweckmäßig, für eine intensive und rasche Durchmischung des Gasstroms mit der flüssigen Phase zu sorgen. Dabei ist es vorteilhaft, den Perfluorolefinstrom in möglichst feinverteilter Form, beispielsweise durch Verwendung von Düsen oder Fritten, einzuleiten und/oder die Elektrolytflüssigkeit durch Rühren oder Umpumpen kräftig zu bewegen.

In dem — bevorzugten — Falle der Verwendung von Tetrafluorethylen als Perfluorolefin kann dessen Partialdruck im allgemeinen zwischen etwa 0,1 und 10 bar, vorzugsweise zwischen etwa 0,3 und 3,0 bar betragen. Zur Erzeugung der im unteren Teil dieses Bereichs liegenden Partialdrücke kann die Zugabe des Tetrafluorethylens gegebenenfalls unter Zugabe eines Inertgases wie z. B. Stickstoff erfolgen. Die Erzeugung höherer Partialdrücke kann gegebenenfalls durch Anwendung eines Überdrucks erreicht werden.

Im Falle des Tetrafluorethylens als Perfluorolefin läßt sich die Bildung unerwünschter Feststoffe etwa durch Zugabe von Jod praktisch völlig unterdrücken, wobei Mengen von etwa 10 bis 100 ppm Jod, bezogen auf die Elektrolytmenge, im allgemeinen ausreichend sind.

Im Falle der anderen Perfluorolefine erfolgt während der Umsetzung mit Peroxodisulfuryl-difluorid kaum Polymerisation.

Die angewandten Stromdichten betragen im allgemeinen zwischen etwa 2 und 200 $mA \cdot cm^{-2}$, vorzugsweise etwa 30 bis 100 $mA \cdot cm^{-2}$.

Für das Verfahren sind im allgemeinen vor Beginn der Elektrolyse keine gesonderten Aktivierungs- oder Formierungsoperationen der Elektroden und des Elektrolyts erforderlich. Die Elektrolyse wird durch Einschalten des Stromflusses in der Elektrolysevorrichtung in Gang gesetzt und dann das jeweilige Perfluorolefin eingeleitet.

Auch diese bevorzugte Ausführungsart des erfindungsgemäßen Verfahrens kann im Prinzip sowohl

diskontinuierlich als auch kontinuierlich erfolgen.

Bei der diskontinuierlichen Verfahrensweise wird die Elektrolyse nach einem gewissen Ladungsdurchgang – zweckmäßigerweise von etwa 0,1 bis 0,7 F/Mol der im Elektrolyt ursprünglich enthaltenen Fluorsulfonsäure – beendet. Da die verfahrensgemäß erhältlichen bzw. erhaltenen $\alpha,\omega$-Bis-fluorsulfato-perfluoralkane eine abnehmende Löslichkeit mit steigender Kettenlänge in dem bei der geschilderten bevorzugten Ausführungsform verwendeten Elektrolytsystem besitzen, werden insbesondere die längerkettigen Reaktionsprodukte im Laufe der Reaktion als flüssige Phase aus dem Elektrolyten abgeschieden.

Nach Beendigung des Elektrolyseansatzes können die Reaktionsprodukte entweder durch Destillation oder vorzugsweise durch Scheiden der abgesonderten Fluor-organischen Phase abgetrennt werden. Die bei der Scheide-Methode erhaltene Elektrolytphase wird vorzugsweise nach Ergänzung mit frischer Fluorsulfonsäure für einen nachfolgenden Ansatz wieder verwendet. Auch eine mehrmalige Wiederverwendung der Elektrolytphase ist möglich.

Die Abtrennbarkeit der Reaktionsprodukte durch Scheiden und die Regenerierfähigkeit der Elektrolytphase ermöglichen auch die kontinuierliche Durchführung des Verfahrens durch jedem Fachmann geläufige Operationen.

Die Isolierung und Reindarstellung der Verfahrensprodukte erfolgt in an sich bekannter Weise. Die $\alpha,\omega$-Bis-fluorsulfatoperfluoralkane werden daher nach der Abtrennung aus dem Elektrolysegemisch zur Abtrennung von Elektrolytresten zunächst ein- oder mehrmals mit Wasser und/oder Natriumbicarbonatlösung neutral gewaschen und mit einem nichtbasischen Trockenmittel, beispielsweise Natriumsulfat oder einem Molekularsieb, getrocknet. Dabei kann es vorteilhaft sein, vor der Wäsche eine einfache Destillation unter vermindertem Druck oder eine Filtration des Elektrolyseaustrags zur Abtrennung geringer Mengen fester Nebenprodukte durchzuführen, welche die Separation der Reaktionsprodukte von den Waschflüssigkeiten behindern.

Die Reaktionsprodukte werden nach Wäsche und Trocknung durch eine fraktionierte Destillation in die einzelnen Komponenten aufgetrennt, wobei entweder bei atmosphärischem Druck oder – bei der Destillation von längerkettigen Verbindungen – vorzugsweise unter vermindertem Druck von etwa 10 bis 100 mbar gearbeitet wird.

Es ist schließlich auch möglich, das erfindungsgemäße Verfahren in Verbindung mit der elektrochemischen Herstellung des Peroxodisulfuryl-difluorids als »ex cell«-Prozeß durchzuführen, d. h. das jeweilige Perfluorolefin mit dem anodisch gebildeten Peroxodisulfuryl-difluorid außerhalb der elektrochemischen Zelle in einem mit dieser verbundenen separaten Reaktor umzusetzen. Dabei ist dann ein entsprechender externer Elektrolytkreislauf vorzusehen – zweckmäßig derart, daß dem mit der Elektrolysezelle gekoppelten Reaktor ein Peroxodisulfuryl-difluorid-haltiger (Teil-)Strom des Elektrolyten zugeführt und ein an Peroxodisulfuryl-difluorid verarmter Flüssigkeitsstrom wieder in die Elektrolysezelle zurück geführt wird.

Nach dem erfindungsgemäßen Verfahren werden in hoher Selektivität und Ausbeute (bis etwa 75% d. Th., bezogen auf das Ausgangs-Perfluorolefin) die entsprechenden Perfluorolefin-peroxodisulfuryldifluorid-2 : 1-Addukte erhalten. Im Falle des Tetrafluorethylens als Ausgangs-Perfluorolefin ist dies das 1,4-Bis-fluorsulfato-perfluorbutan:

$$FSO_2{-}O{-}CF_2{-}CF_2{-}CF_2{-}CF_2{-}O{-}SO_2F\,;$$

im Falle des Hexafluorpropens als Ausgangs-Perfluorolefin ist das 2 : 1-Addukt das 1,4-Bis-fluorsulfato-2,3-bis(trifluormethyl)-butan:

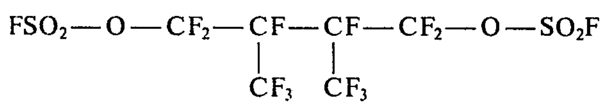

Die – zumindest theoretisch – möglichen Isomeren dieser Verbindung entstehen hier nur in untergeordnetem Maß.

Entsprechend ist die Formel des Verfahrenshauptproduktes, wenn man von höheren perfluorierten $\alpha$-Olefinen ausgeht:

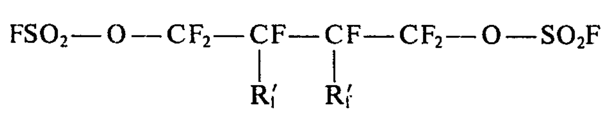

worin $R_f' =$ Perfluoralkylrest mit 2–8 C-Atomen.

Nebenprodukte des erfindungsgemäßen Verfahrens sind in erster Linie die entsprechenden 1 : 1-, 3 : 1-, 4 : 1- und 5 : 1-Addukte aus Perfluorolefin und Peroxodisulfuryl-difluorid. Von diesen sind u. a. die Tetrafluorethylen-peroxodisulfuryl-difluorid-Addukte der Formel

$$FSO_2{-}O{-}(CF_2{-}CF_2)_n{-}O{-}SO_2F\,,$$

worin n = ganze Zahlen von 3–5, neue Verbindungen. Das vorerwähnte Hexafluorpropen-peroxodisulfuryl-difluorid-2 : 1-Addukt der vorgenannten Struktur ist in der Arbeit von C. G. Krespan a. a. O. nicht namentlich erwähnt.

Die nach dem erfindungsgemäßen Verfahren erhaltenen $\alpha,\omega$-Bisfluorsulfato-perfluoralkane werden verwendet, wie dies eingangs in allgemeiner Weise für derartige Verbindungen beschrieben ist. Zusätzlich (und bevorzugt) werden die Verbindungen verwendet zur Herstellung der entsprechenden $\omega$-Fluorsulfatoperfluoralkansäureester nach dem Verfahren der gleichzeitig eingereichten europäischen Patentanmeldung Nr. 82 106 201.5 (Publ. Nr. 0 070 485). Dabei werden die $\alpha,\omega$-Bis-fluorsulfato-perfluoralkane in Gegenwart katalytischer bis etwa äquimolarer Mengen eines oder mehrerer Alkalifluoride und/oder Alkalihydrogenfluoride sowie in Gegenwart einer mindestens äquimolaren Menge eines Alkohols ROH (R = Alkylrest) und gegebenenfalls in Gegenwart auch eines inerten, die Alkalifluoride und/oder -hydrogenfluoride nicht lösenden Lösungsmittels (z. B. Methylenchlorid) umgesetzt. Am Beispiel des 1,4-Bis-fluorsulfatoperfluorbutans stellt sich diese Reaktion wie folgt dar:

$$FSO_2—O—CF_2—CF_2—CF_2—CF_2—O—SO_2F + ROH$$

$$\xrightarrow[\text{-hydrogenfluorid(e)}]{\text{Alkalifluorid(e) und/oder}} FSO_2—O—CF_2—CF_2—CF_2—COOR + SO_2F_2 + HF$$

Die $\omega$-Fluorsulfato-perfluoralkansäureester können dann z. B. nach dem Verfahren der deutschen Patentanmeldung P 30 34 549 durch Zersetzung in Gegenwart nur katalytischer Alkalifluoridmengen und in Abwesenheit von Lösungsmitteln zu den entsprechenden Perfluor-dicarbonsäureesterfluoriden weiterverarbeitet werden, was sich anhand des Beispiels des $\omega$-Fluorsulfato-perfluorbutansäureesters formelmäßig wie folgt wiedergeben läßt:

$$FSO_2—O—CF_2—CF_2—CF_2—COOR$$

$$\xrightarrow{\text{Alkalifluorid}} FOC—CF_2—CF_2—COOR + SO_2F_2$$

Aus den so erhaltenen Perfluoralkan-dicarbonsäurefluoridestern werden dann auf dem anfangs skizzierten Weg (Umsetzung mit Hexafluorpropenepoxid, $KOH/H_2O$, Abspaltung von KF und $CO_2$) die entsprechenden perfluorierten Vinylether mit noch einer Estergruppe am anderen Molekülende hergestellt. Diese Vinylether sind – wie anfangs erwähnt – wichtige Monomere für die Herstellung von Ionenaustauschermassen etc.

Wegen der Einfachheit der Ausgangsprodukte und der Verfahrensweise sowie der hohen Selektivität und Produktausbeute stellt das erfindungsgemäße Verfahren einen erheblichen Fortschritt auf diesem Gebiet dar. Das Verfahren und die Verfahrensprodukte ermöglichen einen – verglichen mit den bisherigen Möglichkeiten – einfacheren und wirtschaftlicheren Zugang insbesondere zu den technisch bedeutenden perfluorierten Vinylethern mit noch einer Estergruppe am anderen Molekülende.

Die Erfindung wird nun durch die nachstehenden Beispiele, welche die bevorzugte Ausführungsform des erfindungsgemäßen Verfahrens (in einer Elektrolysezelle) illustrieren, näher erläutert.

## Beispiel 1

Die Elektrolysezelle besteht aus einer labormäßigen Becherglaszelle von 65 mm Durchmesser und 250 mm Höhe, die mit einem Kühlmantel versehen ist. Auf dem Boden des Gefäßes befindet sich ein 30 mm langer PTFE (Polytetrafluorethylen)-ummantelter Magnetrührstab. Eine plattenförmige Anode von 55 mm Breite und 3 mm Dicke aus glasartigem Kohlenstoff (Sigradur®-K, Hersteller: Sigri Elektrographit GmbH, D-8901 Meitingen), welche am Deckel der Zelle befestigt ist, taucht senkrecht bis etwa 20 mm über den Boden in das Gefäß ein. Beiderseits und parallel zur Anodenplatte im Abstand von ca. 25 mm befindet sich je ein 10 mm breiter Streifen aus Platinblech, der ebenfalls am Zelldeckel befestigt ist und als Kathode dient. Ein Gaseinleitungsrohr mit einer auf etwa 0,5 mm verjüngten Austrittsöffnung reicht bis 20 mm über den Boden der Zelle. Die Vorrichtung besitzt ferner einen Trockeneis-Kühler, ein Thermometer und elektrische Anschlüsse an eine Gleichstromquelle.

Zur Herstellung der Elektrolytlösung werden 14,6 g (0,25 Mol) pulverisiertes Natriumchlorid mit 700 g Fluorsulfonsäure (technisch, Kp. 60°C, $d_4^{20}$ 1,73) versetzt, wobei die Hauptmenge Chlorwasserstoff entweicht. Anschließend wird die Lösung mit trockenem Stickstoff gespült. Sodann leitet man einen Strom von ca. 5 l · $h^{-1}$ Tetrafluorethylen unter kräftigem Rühren ein und elektrolysiert 10 Stunden bei 8 A und einer Temperatur von 25–35°C. Die Zellspannung steigt dabei von 12 auf 17 V an. Nach Beendigung der Elektrolyse werden 340 g flüssige Reaktionsprodukte als Unterphase abgetrennt und die Elektrolytphase mit 160 g frischer Fluorsulfonsäure ergänzt und für den folgenden Ansatz wiederverwendet.

Nach Durchführung eines zweiten Ansatzes in der vorstehend beschriebenen Ausführung lassen sich 520 g flüssige Reaktionsprodukte abtrennen. Die Elektrolysephase wird mit 320 g frischer Fluorsulfonsäure ergänzt. Drei weitere Ansätze jeweils unter Recyclisierung der Elektrolytphase verlaufen mit den gleichen Ergebnissen wie der zweite Ansatz.

Die vereinigten rohen Elektrolyseausträge (2375 g) werden zur Abtrennung geringer Feststoff-Anteile einer einfachen Destillation bei 100–10 mbar unterzogen, wobei 2310 g Destillat erhalten werden Das Destillat wird zunächst mit Wasser und anschließend mit Natriumhydrogencarbonat-Lösung neutral gewaschen und anschließend über einem Molekularsieb 4 Å getrocknet. Das getrocknete Rohprodukt (2000 g) besitzt nach gaschromatographischer Analyse folgende Zusammensetzung (Flächen-%):

7,8% $FSO_2-O-CF_2-CF_2-O-SO_2F$
76,0% $FSO_2-O-(CF_2-CF_2)_2-OSO_2F$
12,9% $FSO_2-O-(CF_2-CF_2)_3-O-SO_2F$
2,1% $FSO_2-O-(CF_2-CF_2)_4-O-SO_2F$
0,5% $FSO_2-O-(CF_2-CF_2)_5-O-SO_2F$ .

Die Isolierung und Reindarstellung der Komponenten n = 1, 2 und 3 erfolgte durch fraktionierte Destillation des Gemisches über eine 1,2-m-Füllkörperkolonne mit Raschigringen unter Zugabe von 10 g pulverisiertem Calciumoxid. Folgende Fraktionen wurden erhalten:

| Fraktion | Bezeichnung | Menge | Gehalt nach GC (Flächen-%) |
|---|---|---|---|
| Kp bis 105°C | $FSO_2-O-CF_2-CF_2-OSO_2F$ | 123 g | 89,4 |
| Kp 105–137°C | Zwischenlauf I | 115 g | – |
| Kp 137–138°C | $FSO_2-O-(CF_2-CF_2)_2-O-SO_2F$ | 1367 g | 99,8 |
| Kp 138–168°C | Zwischenlauf II | 70 g | – |
| Kp 168–174°C | $FSO_2-O-(CF_2-CF_2)_3-OSO_2F$ | 176 g | 97,6 |
| – | Rückstand | 137 g | – |

Die Isolierung und Reindarstellung der Komponenten n = 4 und 5 erfolgte durch fraktionierte Destillation der vereinigten Rückstände (740 g) aus mehreren der vorstehend beschriebenen Destillationen. Unter Verwendung 1-m-Vigreuxkolonne bei einem Druck von 14 mbar wurden folgende Fraktionen erhalten:

| Fraktion | Bezeichnung | Menge | Gehalt nach GG (Flächen-%) |
|---|---|---|---|
| $Kp_{14}$ bis 90°C | Vorlauf | 109 g | – |
| $Kp_{14}$ 90–93°C | $FSO_2-O-(CF_2-CF_2)_4-OSO_2F$ | 431 g | 96,9 |
| $Kp_{14}$ 94–115°C | Zwischenlauf | 49 g | – |
| $Kp_{14}$ 115–117°C | $FSO_2-O-(CF_2-CF_2)_5-O-SO_2F$*) | 101 g | 96,8 |
| – | Rückstand | 47 g | – |

*) Fp 39–40°C.

$^{19}F$-NMR ($CDCl_3$):

$FSO_2-O-(CF_2-CF_2)_2-O-SO_2F$
+50,9 (t, 2F, $-O-SO_2F$, Y = 8 Hz)
−83,4 (m, 4F, $-O-CF_2-$)
−125,0 (m, 4F, $-CF_2-$)
$FSO_2-O-(CF_2-CF_2)_3-O-SO_2F$
+50,9 (t, 2F, $-O-SO_2F$, Y = 8 Hz)
−83,2 (m, 4F, $-O-CF_2$)
−122,2 (m, 4F, $-CF_2-$)
−124,8 (m, 4F, $-CF_2-$)
$FSO_2-O-(CF_2-CF_2)_4-O-SO_2F$
+50,8 (t, 2F, $-O-SO_2F$, Y = 8 Hz)
−83,4 (m, 4F, $-O-CF_2-$)
−122,2 (m, 4F, $-CF_2-$)
−124,9 (m, 8F, $-CF_2-$)
$FSO_2-O-(CF_2-CF_2)_5-O-SO_2F$
+50,6 (t, 2F, $-O-SO_2F$, Y = 8 Hz)
−83,5 (m, 4F, $-O-CF_2-$)
−122,2 (m, 4F, $-CF_2-$)
−125,0 (m, 12F, $-CF_2-$)

Die Peroxodisulfuryl-difluorid-Konzentration in der flüssigen Elektrolytphase betrug bei allen Ansätzen dieses Beispiels in der Anfangsphase des 1. Ansatzes ca. 0,06 Mol/l und im weiteren Verlauf etwa 0,015 Mol/l (Bestimmungsmethode: 2 ml Elektrolyt werden in eine mit Eis versetzte KJ-Lösung gegeben und das ausgeschiedene Jod mit Thiosulfatlösung bestimmt).

Beispiel 2

Man verwendet die in Beispiel 1 beschriebene Elektrolysevorrichtung.

Zur Herstellung des Elektrolyten bereitet man eine Lösung aus 37,2 g (0,5 Mol) Kaliumchlorid und 750 g Fluorsulfonsäure und entfernt den Chlorwasserstoff durch Ausblasen mit Stickstoff. Ein unter Rühren eingeleiteter Strom von gasförmigem Hexafluorpropylen (ca. 7—9 $l \cdot h^{-1}$) ist so bemessen, daß stets ein Überschuß an Hexafluorpropylen vorhanden ist (Rückfluß). Bei einer Stromstärke von 8A und einer Temperatur von 25—35°C wird bis zu einem Ladungsdurchgang von 68 Ah elektrolysiert. Die Zellspannung steigt dabei von 14 auf 19 Volt an. Nach Beendigung der Elektrolyse werden 520 g fluororganische Reaktionsprodukte als Unterphase abgetrennt, der Elektrolyt mit 250 g frischer Fluorsulfonsäure ergänzt und für den folgenden Ansatz wiederverwendet. Nach Durchführung des zweiten Ansatzes bis zu einem Ladungsdurchgang von 73 Ah werden 650 g fluororganische Phase abgetrennt und der Elektrolyt mit 280 g frischer Fluorsulfonsäure ergänzt. Nach Durchführung von insgesamt 15 Elektrolyseansätzen unter Wiederverwendung der Elektrolytphase wurden 9140 g fluororganische Reaktionsprodukte erhalten.

Der Anteil der 2 : 1-Addukte in den fluororganischen Reaktionsprodukten beträgt nach GC im 1. bis 5. Ansatz durchschnittlich 84,5 Fl.-%, im 6. bis 10. Ansatz durchschnittlich 85,1 Fl.-% und im 11. Ansatz bis 15. Ansatz durchschnittlich 88,5 Fl.-%.

Die vereinigten rohen Elektrolyseausträge des 1. bis 5. Ansatzes (3040 g) werden mehrmals mit Wasser und anschließend mit Natriumhydrogencarbonat-Lösung gewaschen und über Molekularsieb 5Å getrocknet. Das getrocknete Rohgemisch (2855 g) wird nach Zugabe von 10 g Calciumoxid durch fraktionierte Destillation unter vermindertem Druck von 290 mbar über eine 1,2-m-Füllkörperkolonne mit Raschig-Ringen in folgende Fraktionen aufgetrennt:

| Fraktion | Bezeichnung | Menge | Gehalt (Flächen-%) |
|---|---|---|---|
| $Kp_{290}$ bis 124°C | Vorlauf | 360 g | — |
| $Kp_{290}$ 124—125°C | $[FSO_2-O-CF_2-CF(CF_3)]_2$ | 2241 g | 71,4*) |
| — | Rückstand | 176 g | — |

*) Die Abtrennung der übrigen 2 : 1-Addukte durch fraktionierte Destillation gelingt nicht. Die Nebenprodukte stören jedoch bei verschiedenen nachfolgenden Umsetzungen nicht.

$^{19}F$ NMR ($CDCl_3$):

$FSO_2-O-CF_2-CF(CF_3)-CF(CF_3)-CF_2-O-SO_2F$
Diastereomeren-Gemisch
+51,0 (m, 4F, $-O-SO_2F-$)
−69,0 (m, 6F, $-CF_3$)
−69,5 (m, 6F, $-CF_3$)
−73,0 (m, 4F, $-CF_2-$)
−73,5 (m, 4F, $-CF_2-$)
−176,0 (m, 4F, −CF)

Die Peroxodisulfuryl-difluorid-Konzentration in der flüssigen Elektrolytphase betrug in der Anfangsphase des 1. Ansatzes etwa 0,035 Mol/l und im weiteren Verlauf dann etwa 0,012 Mol/l (Bestimmung wie in Beispiel 1).

## Patentansprüche

1. Verfahren zur Herstellung von $\alpha,\omega$-Bis-fluorosulfatoperfluoroalkanen durch Umsetzung von perfluorierten $\alpha$-Olefinen mit Peroxodisulfuryl-difluorid $FSO_2-O-O-SO_2F$ hauptsächlich zu den 2 : 1-Addukten, dadurch gekennzeichnet, daß man perfluorierte $\alpha$-Olefine der Formel

$$CF_2{=}CF-R_f,$$

worin $R_f$ = F oder Perfluoralkyl mit 1—8 C-Atomen, vorzugsweise F oder $CF_3$, insbesondere nur F, bedeutet, in eine Lösung des Peroxodisulfuryl-difluorids in einem inerten Lösungsmittel einleitet, wobei man die Konzentration des Peroxodisulfuryl-difluorids in der Lösung innerhalb des Konzentrationsbereiches von 0,005 bis 0,2, vorzugsweise von 0,01 bis 0,1 Mol/l, im wesentlichen konstant hält.

2. Verfahren nach Anspruch 1, dadurch gekennzeichnet, daß man die perfluorierten $\alpha$-Olefine in die flüssige Phase einer Elektrolysezelle einleitet, in welcher durch Elektrolyse einer Lösung von Alkalifluorsulfat in Fluorsulfonsäure Peroxodisulfuryl-difluorid gebildet und im Ausmaß von dessen Verbrauch laufend nachgeliefert wird.

## Claims

1. A process for the preparation of $\alpha,\omega$-bis-fluorosufaltoperfluoroalkanes by reacting perfluorinated $\alpha$-olefins with peroxodisulfuryl difluoride $FSO_2-O-O-SO_2F$ to yield chiefly the 2 :1-adducts, characterized in passing perfluorinated $\alpha$-olefins of the formula

$$CF_2{=}CF-R_f,$$

in which $R_f$ denotes F or perfluoroalkyl containing 1—8 C atoms, preferably F or $CF_3$, and in particular only F into a solution of peroxodisulfuryl difluoride in an inert solvent, the concentration of the peroxodisulfuryl difluoride in said solution being kept substantially constant within a concentration range of 0.005—0.2, preferably 0.01—0.1 mole/1.

2. The process as claimed in claim 1, characterized in passing the perfluorinated $\alpha$-olefins into the liquid phase or an elektrolytic cell in which peroxodisulfuryl difluoride is formed by electrolysis of a solution of an alkali metal fluorosulfonate in fluorosulfonic acid, and is supplemented continuously at the rate at which it is consumed.

## Revendications

1. Procédé de préparation d'$\alpha,\omega$-bis-fluorosulfato-perfluoroalcanespar réaction d'$\alpha$-oléfines perfluorées avec le difluorure de peroxodisulfuryle $FSO_2-O-O-SO_2F$ donnant surtout des composés d'addition 2 : 1, procédé caractérisé en ce qu'on introduit des $\alpha$-oléfines perfluorées de formule:

$$CF_2{=}CF-R_f,$$

(dans laquelle $R_f$ représente F ou un groupe perfluoralkyle comportant 1 à 8 atomes de carbone, et represente de préférence F ou $CF_3$, en particulier F seulement) dans une solution du difluorure de peroxodisulfuryle dans un solvant inerte, en maintenant essentiellement constante la concentration du difluorure de peroxodisulfuryle dans les solutions, à l'intérieur de l'intervalle de concentration compris entre 0,005 et 0,2, avantageusement entre 0,01 et 0,1 mole/litre.

2. Procédé selon la revendication 1, caractérisé en ce qu'on introduit des $\alpha$-oléfines perfluorées dans la phase liquide d'une cellule d'électrolyse, dans laquelle on forme par électrolyse d'une solution de fluorosulfate de métal alcalin dans l'acide fluorosulfonique, du difluorure de peroxodisulfuryle, et on introduit ensuite continuellement à mesure de sa consommation.